# EUROPEAN PATENT SPECIFICATION

(11) **EP 2 778 172 B1**
(45) Date of publication and mention of the grant of the patent: **01.02.2017**
(21) Application number: 14159462.2
(22) Date of filing: 13.03.2014
(51) Int. Cl.: C07H 1/00, C07H 15/252

(54) **Stable crystalline monohydrate of epirubicin hydrochloride and method of production**
Stabiles kristallines Monohydrat aus Epirubicinhydrochlorid und Herstellungsverfahren
Monohydrate cristallin stable d'hydrochlorure d'épirubicine et procédé de production

(30) Priority: 15.03.2013 EP 13159392
(43) Date of publication of application: 17.09.2014
(73) Proprietor: Synbias Pharma AG, 8200 Schaffhausen (CH)
(72) Inventor: Zabudkin, Alexander, 83055 Donetsk (UA); Matvyeyev, Alexey, 83114 Donetsk (UA); Matvienko, Victor, 83114 Donetsk (UA)
(74) Representative: Kopf Westenberger Wachenhausen Patentanwälte PartG mbB

(56) References cited:
- WO-A1-2010/039159
- WO-A1-2012/163508
- WO-A2-2005/004805

## Description

### FIELD OF THE INVENTION

The present invention relates to a stable crystalline monohydrate of epirubicin hydrochloride (i.e., 4'-epidoxorubicin hydrochloride) and a corresponding method of production. More particularly, the invention is directed to a crystalline monohydrate of epirubicin hydrochloride having a water content of 2.7% to 3.5% (w/w), being devoid of residual solvents, and exhibiting a uniform size distribution of the crystalline particles produced.

### BACKGROUND OF THE INVENTION

Anthracyclines represent a class of naturally occurring bioactive compounds derived from the bacterial genus *Streptomyces.* Several anthracyclines, including daunorubicin, idarubicin, doxorubicin, epirubicin, and carminomycin, were clinically demonstrated to be effective antineoplastic agents that can be employed for the treatment of a wide range of cancers including breast cancer, lung cancer, and hematological malignancies such as leukemias and lymphomas. In addition, members of this class of compounds were also shown to be useful in bone marrow transplants and during stem cell transplantation.

Epirubicin is the 4-epimer of doxorubicin and a semi-synthetic derivative of daunorubicin and represented by the following formula:

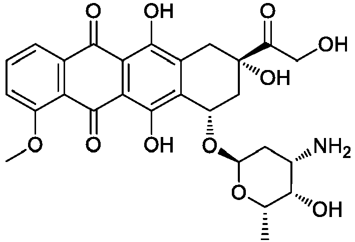

Although the precise mechanisms of its cytotoxic properties have not yet been completely elucidated, it is believed that epirubicin forms complexes with DNA by intercalation of its planar rings between nucleotide base pairs, with consequent inhibition of nucleic acid (DNA and RNA) and protein synthesis, by triggering DNA cleavage via topoisomerase II. Epirubicin and its addition salts, such as epirubicin hydrochloride, are used as a component of adjuvant treatment of breast cancer.

Various methods for the production of epirubicin hydrochloride are currently known in the art. However, amorphous epirubicin hydrochloride (e.g., prepared as described in U.S. Patent 4,861,870) is generally considered as not suitable for pharmaceutical applications due to its significant hygroscopy and chemical instability during storage, with accumulation of the aglycone doxorubicinone.

Certain crystalline polymorphs of epirubicin hydrochloride, being characterized by particular X-ray diffraction patterns, have previously become available and are described *inter alia* in the patent documents: WO 2005/004805 A1; WO 2010/039159 A1; WO 2011/118929 A2; and WO 2012/163508 A1. Although the various X-ray diffraction patterns show a high degree of similarity, the corresponding methods of production are considerably diverse. Notably, the synthesis schemes described in the two first-mentioned documents have subsequently been indicated as not reliably resulting in a crystalline product (cf. WO 2012/163508 A1; paragraphs [0007] and [0069]), and in particular in no product having a uniform particle size distribution, which is to be considered as a key aspect for pharmaceutical applicability.

The crystalline polymorphs of epirubicin hydrochloride described in WO 2005/004805 A1 and WO 2010/039159 A1 (designated "type II") as well as WO 2011/118929 A2 (designated "form A" to "form D") have been shown to exhibit some thermal stability during storage of up to six months.

However, several of the production methods employed in the above patent documents involve the use of significant amounts of solvents. It is well established in the art that anthrycyclines form solvates (cf., e.g., Courseille, C. et al. (1979) Acta Cryst. B35, 764-767). Such solvates are known to show an improved thermal stability as compared to the respective "pure" compound. The above-referenced production schemes do not address the problem of the formation of solvates. Hence, it is not fully apparent whether the thermal stability observed for the above-referenced polymorphs of epirubicin hydrochloride can in fact be assigned to the (pure) compound *per se* or is influenced by the formation of solvates (i.e. the presence of residual solvents in the final product).

Beyond, it is also evident that solvates are more difficult to be controlled for their hygroscopic properties as solvates have an increased tendency to absorb water. The presence of residual solvents (at least in readily detectable amounts) should also have influence on product safety (cf. *inter alia* ICH [International Conference on Harmonization of Technical Requirements for Registration of Pharmaceuticals for Human Use] Guideline Q3C (R5)). For example, the production of crystal forms A, C, and D according to WO 2011/118929 A2 involves the use of class 2 solvents (as defined by ICH), such as acetonitrile and N,N-dimethyl-formamide, whose use in pharmaceutical products should be limited due to their inherent toxicity.

Thus, there is still a need for a crystalline form of epirubicin hydrochloride that overcomes the above limitations, in particular with regard to uniformity of the product, long-term product stability, and product safety.

In particular, there remains a need for a compound having an improved thermal stability and a prolonged shelf-life, while also exhibiting a reduced hydroscopic capacity (i.e. stability against water) and being virtually devoid of residual solvents.

Accordingly, it is an object of the present invention to provide such crystalline form of epirubicin hydrochloride as well as a corresponding method for its production.

### SUMMARY OF THE INVENTION

In one aspect, the present invention relates to a method for producing a crystalline monohydrate of epirubicin hydrochloride, comprising: (a) adding at least a first solvent and at least a second solvent to epirubicin hydrochloride, wherein (i) the first solvent is selected from the group consisting of 1-butanol, 2-butanol, isobutanol, tert-butanol, and mixtures thereof; (ii) the second solvent is selected from the group consisting of 1-propanol, isopropanol, ethanol, and mixtures thereof; and (iii) the volume ratio of the first solvent to the second solvent is in the range between 1:1 and 2:1 (v/v) (b) adjusting in the solution obtained in step (a) the water content to an amount in the range between 8% and 11 % (w/w); and (c) heating the solution obtained in step (b) to a temperature of 70°C to 90°C in order to allow crystallization; wherein the crystalline monohydrate produced has a water content in the range between 2.7% and 3.5% (w/w) and is devoid of residual solvents.

Particularly preferably, the first solvent is 1-butanol and the second solvent is 1-propanol.

In particular embodiments, the method further comprises: heating the mixture of step (a) to a temperature of 40°C to 55°C until complete dissolution of the reaction mixture is accomplished.

In further particular embodiments, the final concentration of epirubicin hydrochloride in the solution of step (b) is in the range between 10 g/l and 100 g/l.

In preferred embodiments, the solution in step (c) is heated to a temperature of 75°C to 85°C.

In specific embodiments, the method further comprises seeding of the solution obtained in step (b) in order to induce crystallization.

In other specific embodiments, the method further comprises purifying the crystals obtained in step (c).

In preferred embodiments, the water content of the crystalline monohydrate of epirubicin hydrochloride produced is in the range between 2.8% and 3.3% (w/w).

In another aspect, the present invention relates to a crystalline monohydrate of epirubicin hydrochloride being produced by the method as described herein above, wherein (i) the mean diameter of the crystal particles produced is in the range between 20 µm and 50 µm, and (ii) the particle size of at least 60%, and particularly of at least 80% of the crystal particles produced is within the range given by the mean diameter ± 20%.

In preferred embodiments, the particle size of at least 60%, and particularly of at least 80% of the crystal particles produced is within the range given by the mean diameter ± 10%.

In particular embodiments, the crystalline monohydrate of epirubicin hydrochloride is further characterized by either one or both of the group consisting of a powder X-ray diffraction pattern comprising peaks at average diffraction angles (2⊖) of 5.1°, 9.1°, 13.6°, 22.1°, 22.5°, and 24.0° (each ± 0.2°) and a thermostability resulting in the production of less than 0.5% (w/w) doxorubicinone after incubation at 100°C for one month according to ICH guidelines.

A further aspect of the present invention relates to the crystalline monohydrate of epirubicin hydrochloride as described herein above for use in the prevention and treatment of cancer.

### BRIEF DESCRIPTION OF THE DRAWINGS

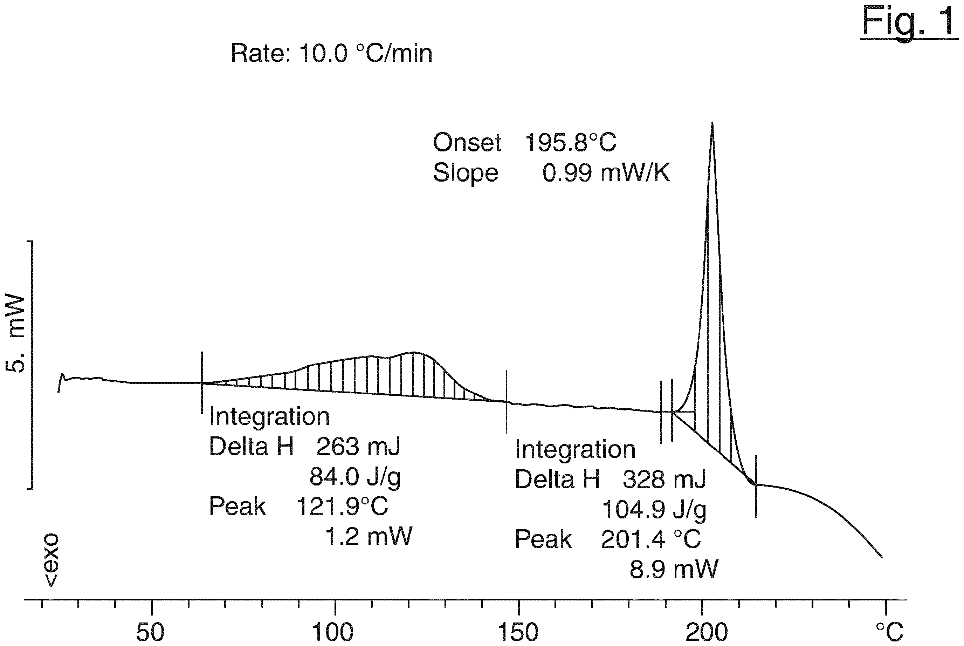

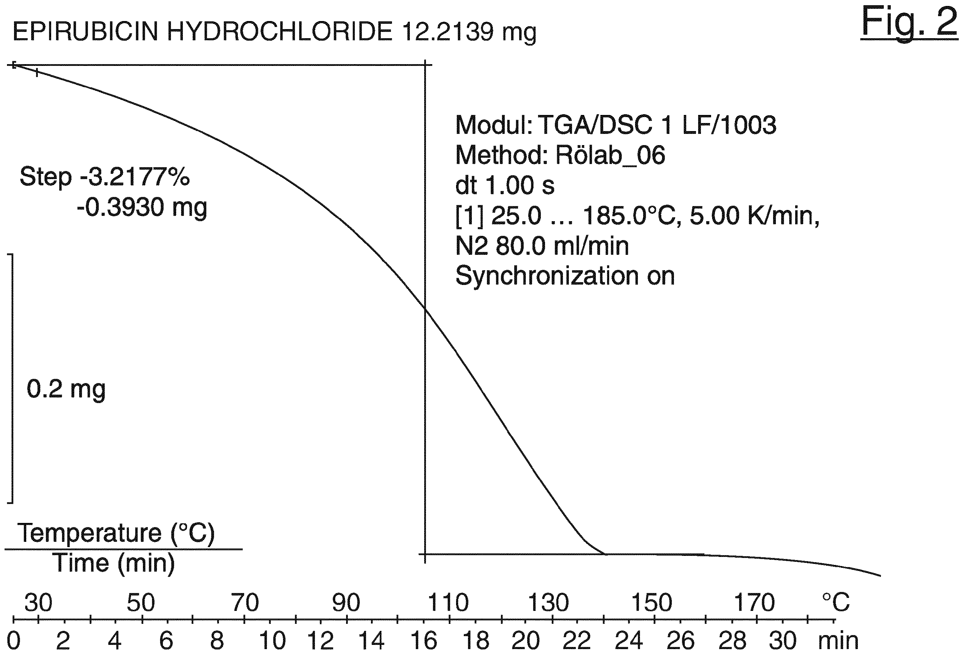

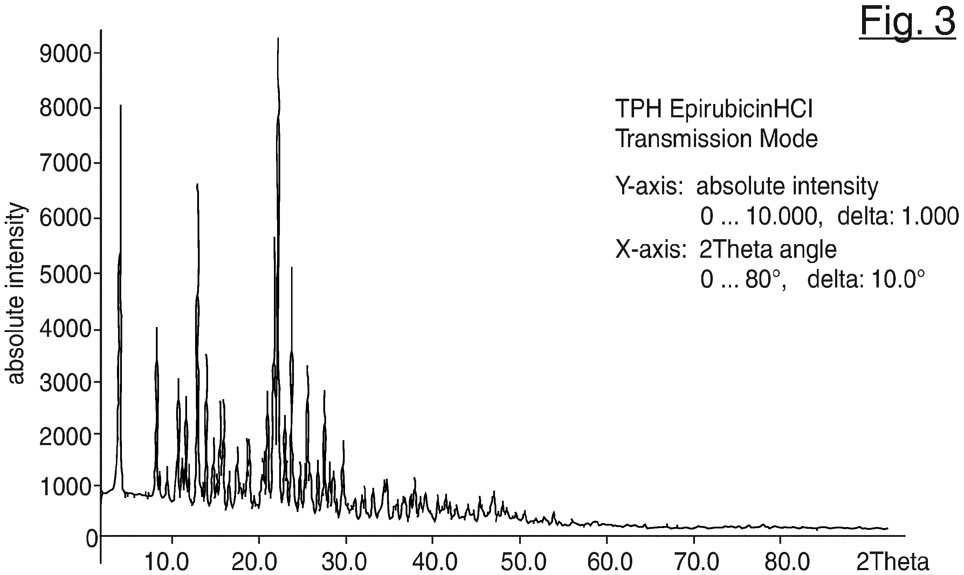

**FIGURES 1-3** illustrate the functional characterization of an exemplary crystalline monohydrate of epirubicin hydrochloride of the present invention, being prepared according to Example 1. **FIG. 1** shows the results of a representative DSC analysis for determining the melting point; **FIG. 2** the results of a representative thermogravimetric analysis for determining the water content; and **FIG. 3** the results of a representative X-ray powder diffraction analysis.

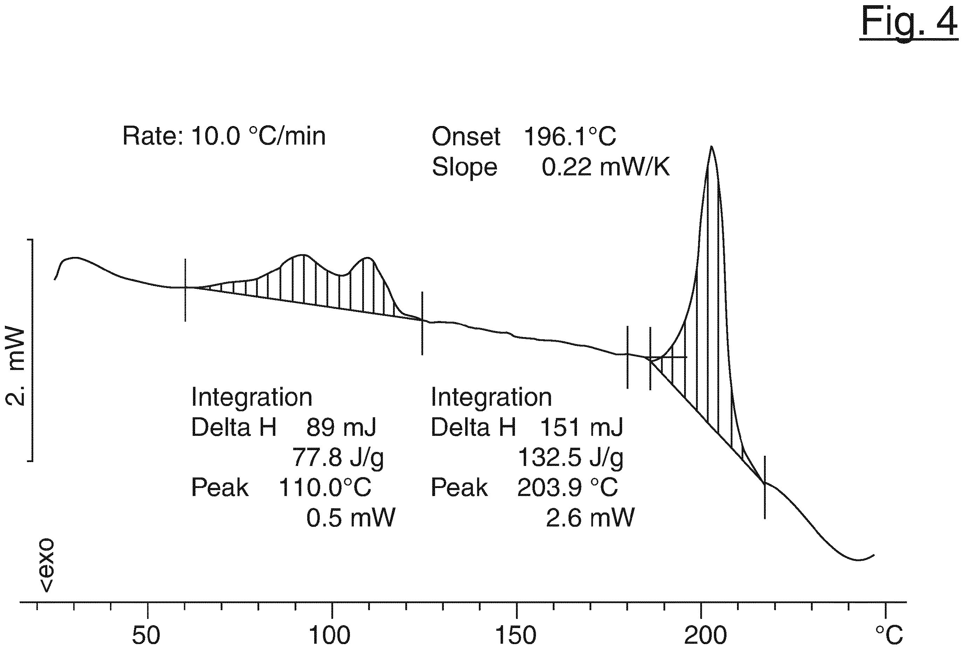

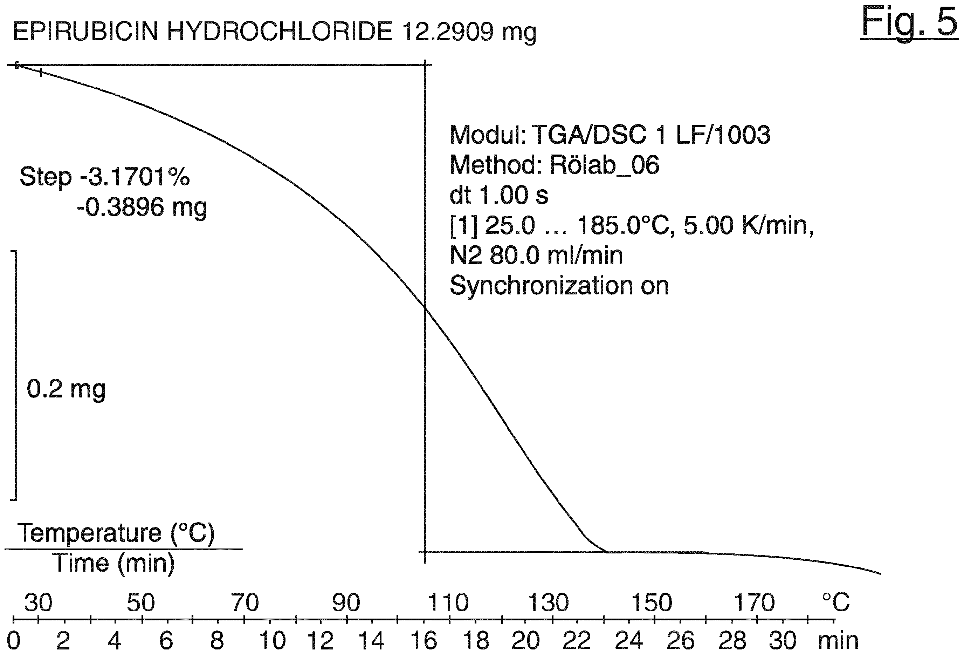

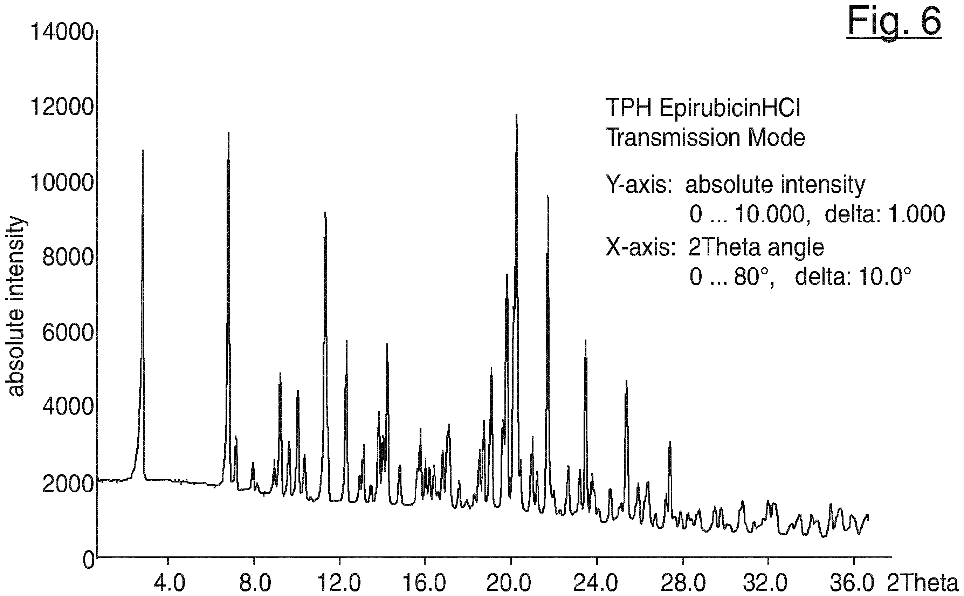

**FIGURES 4-6** illustrate the functional characterization of an exemplary crystalline monohydrate of epirubicin hydrochloride of the present invention, being prepared according to Example 2. **FIG. 4** shows the results of a representative DSC analysis for determining the melting point; **FIG. 5** the results of a representative thermogravimetric analysis for determining the water content; and **FIG. 6** the results of a representative n X-ray powder diffraction analysis.

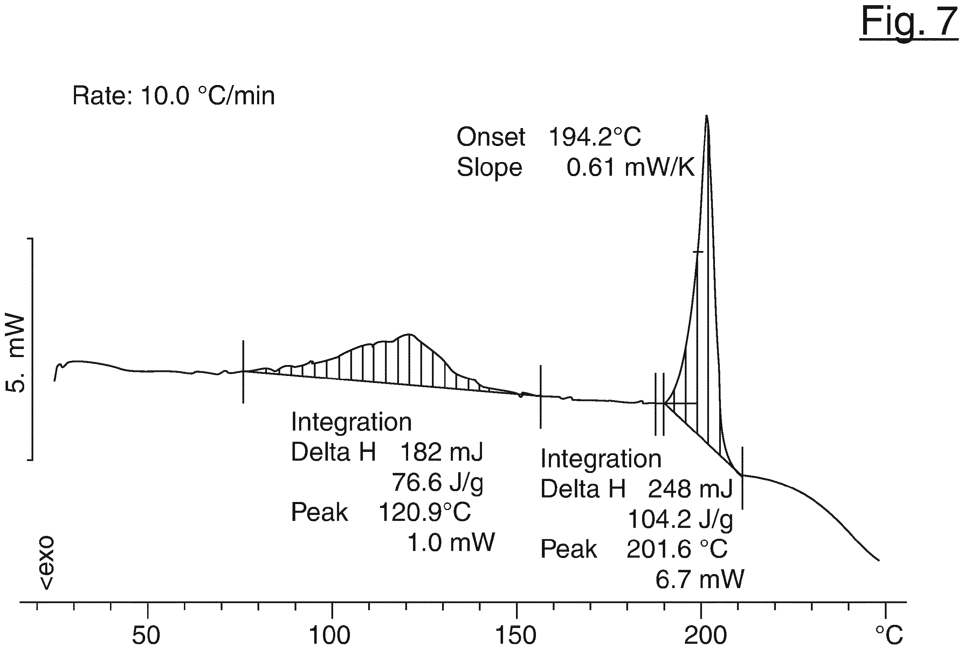

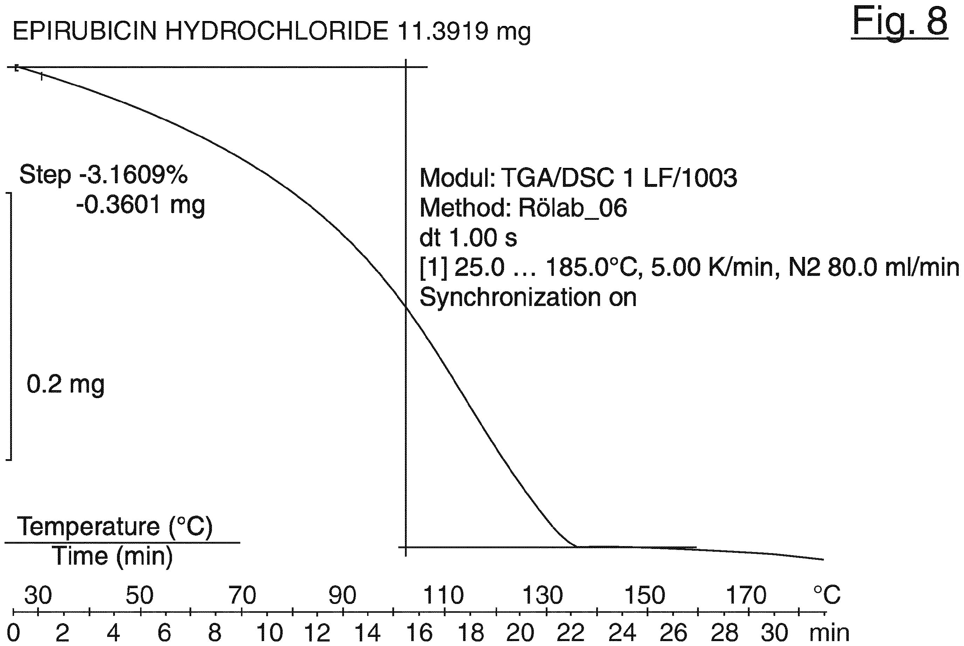

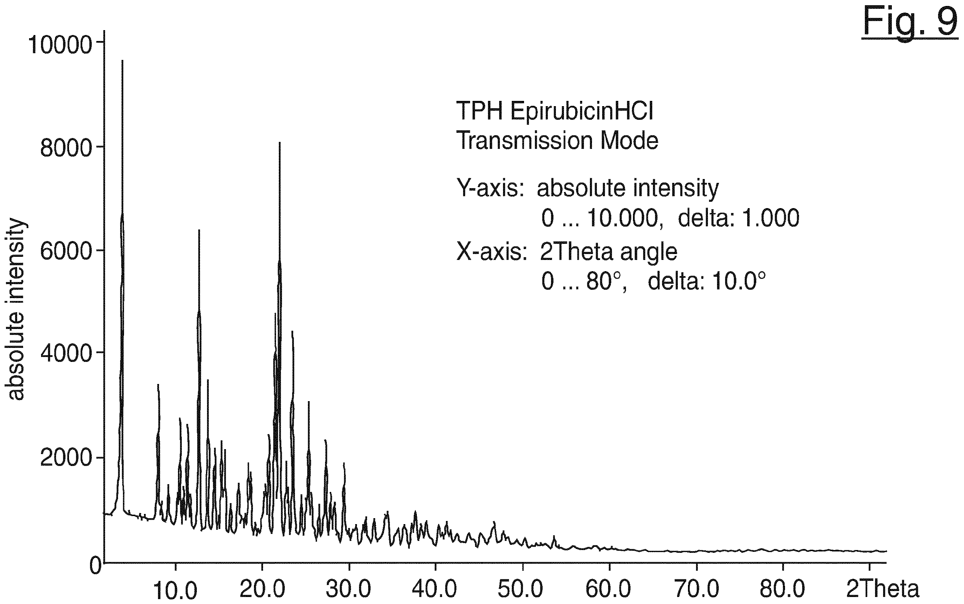

**FIGURES 7-9** illustrate the functional characterization of an exemplary crystalline monohydrate of epirubicin hydrochloride of the present invention, being prepared according to Example 3. **FIG. 7** shows the results of a representative DSC analysis for determining the melting point; **FIG. 8** the results of a representative thermogravimetric analysis for determining the water content; and **FIG. 9** the results of a representative X-ray powder diffraction analysis.

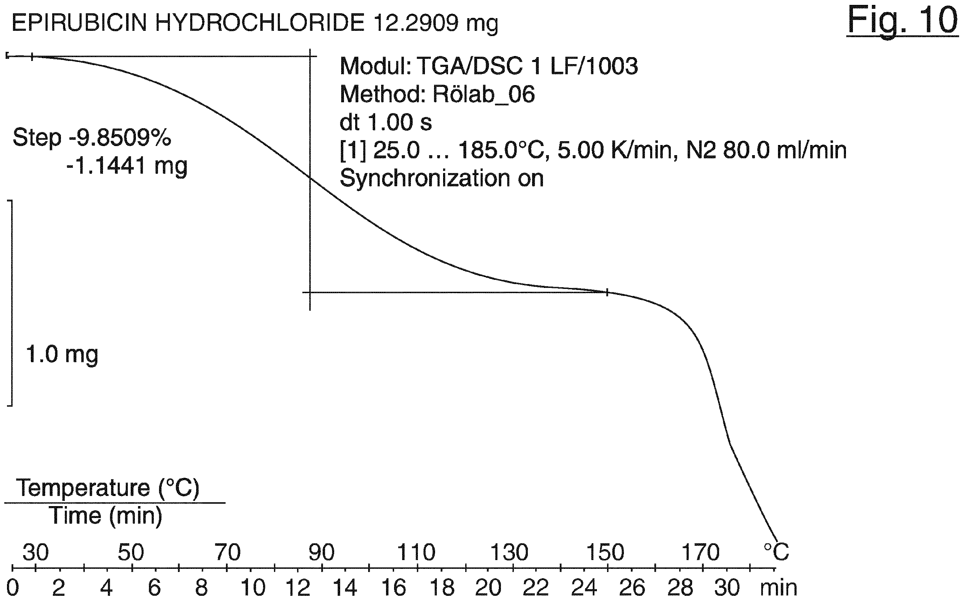

**FIGURE 10** illustrates a representative thermogravimetric analysis for determining the water content of a crystalline trihydrate of epirubicin hydrochloride, being prepared according to Example 3.

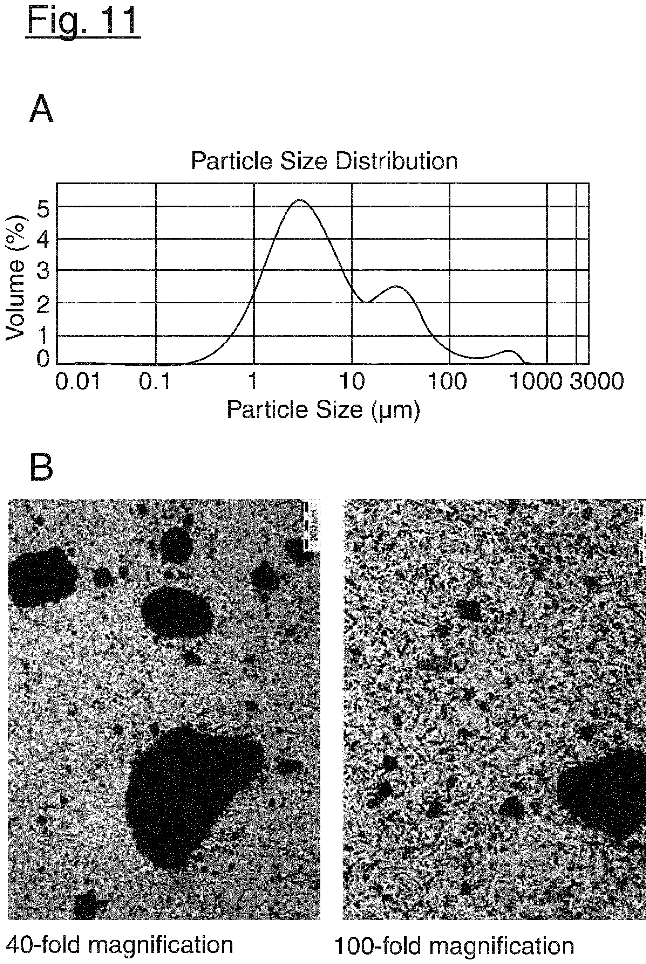

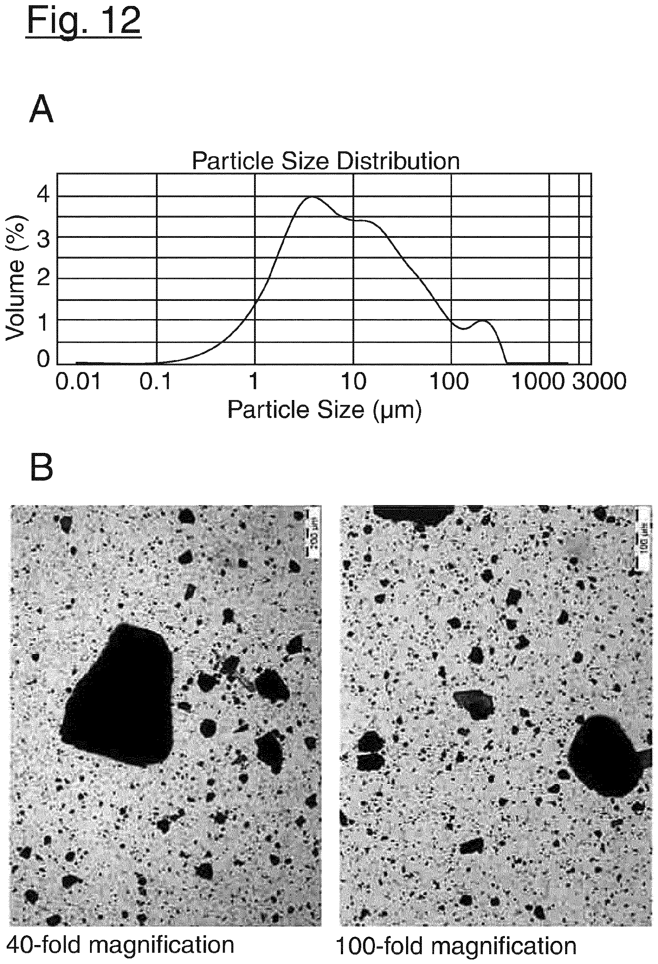

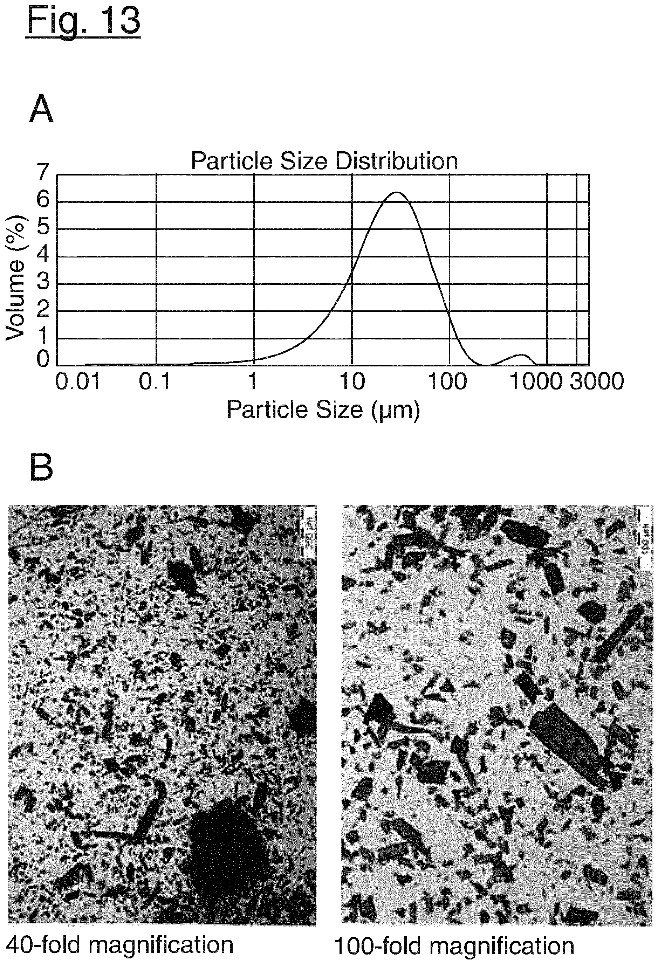

**FIGURES 11-13** illustrate the particle size distributions of different samples of crystalline epirubicin hydrochlorides, as determined by laser diffraction analysis using a Mastersizer 2000 Particle Size Analyzer (Malvern Instruments Ltd., Malvern, United Kingdom). **FIG. 11** shows the results of a representative analysis using a crystalline epirubicin hydrochloride being prepared according to example 1 of patent document WO 2012/163508 A1; **FIG. 12** the results of a representative analysis using a crystalline epirubicin hydrochloride being prepared according to example 2 of patent document WO 2012/163508 A1; and **FIG. 13** the results of a representative analysis using a crystalline monohydrate of epirubicin hydrochloride of the present invention.

### DETAILED DESCRIPTION OF THE INVENTION

The present invention is related to the unexpected finding that the provision of epirubicin hydrochloride in form of a crystalline monohydrate having a water content in the range between 2.7% and 3.5% (w/w) combines an improved thermal stability and a long-term shelf-life with the provision of a highly uniform product, advantageous hygroscopic properties as well as an superior product safety (due to the absence of residual solvents). The adjustment of the water content of the solvent mixture prior to crystallization to an amount in the range between 8% and 11% (w/w) in combination with a comparably high crystallization temperature and a specific combination of solvents, as specified in claim 1, has been found to be a characteristic technical feature in obtaining the crystalline monohydrate.

Without intending to be bound by a particular theory the presence of a certain amount of residual water in the crystal polymorph is considered to interfere with the incorporation of solvent molecules in the crystal structure and/or to displace them from the crystal structure without having any significant adverse effect on the stability of the crystals against heat or water, with the latter effect being particularly surprising.

Notably, when preparing epirubicin hydrochloride in form of a trihydrate by increasing the amount of water present in the solvent mixture, the superior properties identified for the monohydrate form were no longer observed. Particularly, the trihydrate form is considerably less stable against heat and water than the monohydrate form. Accordingly, the superior properties are specifically associated with the monohydrate form of epirubicin hydrochloride. The present invention will be described in the following with respect to particular embodiments and with reference to certain drawings but the invention is to be understood as not limited thereto but only by the appended claims. The drawings described are only schematic and are to be considered non-limiting.

Where the term "comprising" is used in the present description and claims, it does not exclude other elements or steps. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Where an indefinite or definite article is used when referring to a singular noun e.g. "a", "an" or "the", this includes a plural of that noun unless specifically stated otherwise.

In case, numerical values are indicated in the context of the present invention the skilled person will understand that the technical effect of the feature in question is ensured within an interval of accuracy, which typically encompasses a deviation of the numerical value given of ± 10%, and preferably of ± 5%.

Furthermore, the terms first, second, third, (a), (b), (c), and the like in the description and in the claims, are used for distinguishing between similar elements and not necessarily for describing a sequential or chronological order. It is to be understood that the terms used are interchangeable under appropriate circumstances and that the embodiments of the invention described herein are capable of operation in other sequences than described or illustrated herein.

Further definitions of term will be given in the following in the context of which the terms are used. The following terms or definitions are provided solely to aid in the understanding of the invention. These definitions should not be construed to have a scope less than understood by a person of ordinary skill in the art.

In one aspect, the present invention relates to a method for producing a crystalline monohydrate of epirubicin hydrochloride, comprising:
(a) adding at least a first solvent and at least a second solvent to epirubicin hydrochloride, wherein
   (i) the first solvent is selected from the group consisting of 1-butanol, 2-butanol, isobutanol, tert-butanol, and mixtures thereof;
   (ii) the second solvent is selected from the group consisting of 1-propanol, isopropanol, ethanol, and mixtures thereof; and
   (iii) the volume ratio of the first solvent to the second solvent is in the range between 1:1 and 2:1 (v/v);
(b) adjusting in the solution obtained in step (a) the water content to an amount in the range between 8% and 11 % (w/w); and
(c) heating the solution obtained in step (b) to a temperature of 70°C to 90°C in order to allow crystallization;
wherein the crystalline monohydrate produced has a water content in the range between 2.7% and 3.5% (w/w) and is devoid of residual solvents.

For producing the crystalline monohydrate of epirubicin hydrochloride the compound is mixed with at least a first solvent and at least a second solvent. The starting compound epirubicin hydrochloride may be provided as a solid, in amorphous or in any crystalline form. Alternatively, epirubicin hydrochloride may be provided in form of a solution or a suspension, such as an aqueous solution. The compound can be produced by chemical synthesis or by fermentation or is commercially available from various suppliers.

Typically, epirubicin hydrochloride is provided as a solid and then dissolved in water, for example, in a concentration in the range between 10 g/l to 100 g/l. The pH value of this solution may be in the range between 3.0 and 5.0, preferably between 3.5 and 4.5. In particular embodiments, the water is then removed until a gel state of the solution is achieved. For example, removal of water can be accomplished by evaporation (e.g. using a rotor-evaporator) under low pressure and at a temperature of 30°C to 45°C, preferably at a temperature of 35°C to 40°C.

The "at least first solvent", as defined herein, is a linear or branched C₄ alcohol, that is, an alcohol having four carbon atoms, being selected from the group consisting of 1-butanol, 2-butanol, isobutanol, tert-butanol, and mixtures thereof (such as (1-butanol and isobutanol) or (1-butanol, isobutanol, and tert-butanol).

The "at least a second solvent", as defined herein, is a linear or branched C₂ to C₃ alcohol, that is, an alcohol having two or three carbon atoms, being selected from the group consisting of ethanol, 1-propanol, isopropanol, and mixtures thereof (such as (ethanol and 1-propanol) or (1-propanol and isopropanol) or (ethanol, 1-propanol, and isopropanol)).

In specific embodiments, the at least first solvent may be 1-butanol and the second solvent ethanol. Or, the at least first solvent may be tert-butanol and the at least second solvent a mixture of 1-propanol and ethanol.

In particular embodiments, the first solvent is 1-butanol and the second solvent is selected from the group consisting of 1-propanol, isopropanol, ethanol, and mixtures thereof. In other particular embodiments, the first solvent is selected from the group consisting of 1-butanol, 2-butanol, isobutanol, tert-butanol, and mixtures thereof, and the second solvent is 1-pronanol.

In particularly preferred embodiments, the first solvent is 1-butanol and the second solvent is 1-propanol.

In specific embodiments, epirubicin hydrochloride may be mixed with at least a third or at least a forth solvent, wherein the at least third or at least forth solvent are selected from linear or branched C₁ to C₁₀ alcohols, and particularly from linear or branched C₁ to C₆ alcohols.

The at least first solvent and the at least second solvent may be added to the epirubicin hydrochloride simultaneously or sequentially in any order. Typically, the at least first solvent and the at least second solvent are added sequentially, starting with the at least first solvent.

In particular embodiments, after addition of the at least first solvent, the water being present in the mixture is removed (as complete as possible) until a solid precipitate is formed.

Removal of water can be accomplished by evaporation (e.g. using a rotor-evaporator) under low pressure and at a temperature of 30°C to 45°C, preferably at a temperature of 35°C to 40°C.

After addition of the at least second solvent (either simultaneously or sequentially) the resulting mixture is typically incubated, optionally at an elevated temperature, until complete dissolution is accomplished, that is, any solid precipitates being present have been dissolved. To this end, the mixture may be heated (preferably under stirring) to a temperature of 40°C to 55°C, preferably to a temperature of 45°C to 50°C.

In further particular embodiments, the volume ratio of the first solvent to the second solvent is in the range between 1:1 and 2:1 (v/v). In exemplary embodiments, this volume ratio is 1:1, 1.2:1, 1.4:1, 1.6:1, 1.8:1 or 2:1 (v/v).

Subsequently, the water content in the solution obtained after addition of the at least first solvent and the at least second solvent is adjusted to an amount in the range between 8% and 11 % (w/w). This adjustment can be accomplished by the addition of missing water or the removal of excess water, for example by evaporation (cf. above). The water content can be determined by various methods established in the art, for example by Karl-Fischer titration (Fischer, K. (1935) Angew. Chem. 48, 394-396), wherein a coulometric or volumetric titration may be performed, or by thermogravimetric analysis (reviewed, e.g., in Coats, A.W. and Redfern, J.P. (1963) Analyst 88, 906-924).

In other preferred embodiments of the method, the water content in the solution is adjusted to an amount in the range between 8% and 10% (w/w) or between 9% and 11% (w/w), for example 8.0%, 8.2%, 8.4%, 8.6%, 8.8%, 9.0%, 9.2%, 9.4%, 9.6%, 9.8%, 10.0%, 10.2%, 10.4%, 10.6%, 10.8% or 11.0% (w/w).

In particular embodiments, the final concentration of epirubicin hydrochloride in the solution obtained after the adjustment of the water content is in the range between 10 g/l and 100 g/l, for example 10 g/l, 20 g/l, 50 g/l, 80 g/l or 100 g/l. However, it is also possible to use solutions having a higher concentration of epirubicin hydrochloride, for example up to 120 g/l, 150 g/l or 200 g/l.

The pH value of the solution obtained after the adjustment of the water content may be in the range between 2.5 and 6.0 or between 3.0 and 5.5. In preferred embodiments, the pH value is adjusted to a value in the range between 3.5 and 4.5, for example to 4.0.

The solution obtained (i.e. after addition of the at least first solvent and the at least second solvent to epirubicin hydrochloride and complete dissolution) is then heated to a temperature of 70°C to 90°C in order to allow crystallization. Preferably, the solution is heated to a temperature of 75°C to 85°C, or particularly preferably to a temperature of 78°C to 85°C or of 78°C to 82°C, for example 75°C, 76°C, 77°C, 78°C, 79°C, 80°C, 81 °C, 82°C, 83°C, 84°C or 85°C. The mixture is preferably heated under stirring.

The incubation at a temperature of 70°C to 90°C is continued until a solid precipitate (i.e. crystals of epirubicin hydrochloride) has been formed, for example, for up to 1 hour, for up to 2 hours, for up to 4 hours or for up to 6 hours. Stirring may be continued during incubation.

In a specific embodiment, the method of the present invention comprises:
(a) adding at least a first solvent and at least a second solvent to epirubicin hydrochloride, wherein the first solvent is 1-butanol, and the second solvent is 1-propanol, and the first solvent and the second solvent are provided in a volume ratio in the range between 1:1 and 2:1 (v/v);
(b) adjusting in the solution obtained in step (a) the water content to an amount in the range between 8% and 11 % (w/w); and
(c) heating the solution obtained in step (b) to a temperature of 75°C to 85°C in order to allow crystallization;
wherein the crystalline monohydrate produced has a water content in the range between 2.8% and 3.3% (w/w) and is devoid of residual solvents. Particularly, the method further comprises any one or more of the following steps:
(i) heating the mixture of step (a) to temperature of 45°C to 50°C until complete dissolution is accomplished;
(ii) adjusting the pH value in step (b) to a value in the range between 3.5 and 4.5; and
(iii) continuing the incubation of the solution in step (c) at a temperature of 75°C to 85°C until a solid precipitate has been formed.

In specific embodiments, the method further comprises seeding of the solution obtained after the adjustment of the water content in order to induce crystallization. That is, one or more small epirubicin crystals are added to the solution, from which larger crystals are grown.

In other specific embodiments, the method further comprises purifying the crystals obtained during crystallization. Typically, such purification involves the separation of the crystals from the other components of the mixture, which is usually accomplished by filtration or distillation. Furthermore, the purification process may involve a washing step with a suitable washing solution such as an alcohol or a ketone.

In particular embodiments of the methods, the optional purification process comprises a fluid extraction process using a supercritical fluid, that is, a substance at a temperature and pressure above its critical point, where distinct liquid and gas phases do not exist. Supercritical fluids to be used herein include *inter alia* nitrous oxide, propane, ethylene, fluorocarbons, and carbon dioxide, with the latter one being preferred. The fluid extraction process may be performed at a temperature between 25-55°C (e.g., between 40-45°C) and a pressure between 10-60 MPa (e.g., between 40-55 MPa) for a period of 2-4 hours (e.g., 3 hours) at a flow rate in the range between 200-2000 ml/min (e.g., between 500-1000 ml/min).

Finally, the crystals may be dried according to established protocols, optionally under vacuum. For example, the crystals obtained can be air-dried at a temperature of 30°C to 40°C.

Functional characterization of the resulting crystals revealed that they represent a monohydrated form of epirubicin hydrochloride (i.e. one water molecule per molecule of epirubicin hydrochloride) having a water content in the range between 2.7% and 3.5% (w/w). The water content can be determined as described above, for example by Karl-Fischer titration or by thermogravimetric analysis.

In preferred embodiments of the method, the water content of the crystalline monohydrate of epirubicin hydrochloride is in the range between 2.8% and 3.3% (w/w), for example 2.8%, 2.9%, 3.0%, 3.1%, 3.2% or 3.3% (w/w).

Furthermore, the functional characterization of the crystalline monohydrate of epirubicin hydrochloride produced demonstrated that the compound is devoid of residual solvents. The term "devoid of", as used herein, refers to an amount of residual solvents in the crystals being less than 0.5% (w/w), preferably less than 0.3% (w/w) and particularly preferably less than 0.25% (w/w). The amount of residual solvents present in the crystals can be determined by using various established methods, such as gas chromatography.

In another aspect, the present invention relates to a crystalline monohydrate of epirubicin hydrochloride being produced by the method as described herein above, having a water content in the range between 2.7% and 3.5% (w/w), and being devoid of residual solvents, wherein
(i) the mean diameter of the crystal particles produced is in the range between 20 µm and 50 µm; and
(ii) the particle size of at least 60%, and articularly at least 80% of the crystal particles produced is within the range given by the mean diameter ± 20%.

In other words, the crystalline monohydrate of epirubicin hydrochloride produced by the present invention is characterized by a uniform size distribution of the crystal particles, that is, there is no significant deviation of the size of the vast majority of the individual crystal particles present in a preparation from a mean particle diameter (typically calculated as volume weighted mean value). For example, the particle size distribution may be determined by laser diffraction analysis which is well established in the art (e.g., described in the European Pharmacopeia, 7th Edition (2010), 20931). A representative analysis using a crystalline monohydrate of epirubicin hydrochloride of the present invention is shown in

### FIGURE 13.

First, the mean diameter of the crystal particles produced by the method of the present invention is in the range between 20 µm and 50 µm, for example 22 µm, 25 µm, 28 µm, 30 µm, 35 µm, 40 µm or 45µm.

Second, the particle size of at least 60%, at least 70%, and particularly of at least 80% or at least 90% of the crystal particles produced is within the range given by the mean diameter ± 20%. For example, if the mean diameter of the crystal particles produced is 25 µm, then the respective percentage of the crystal particles present in a given preparation have a diameter in the range between 20 µm and 30 µm. In preferred embodiments, the particle size of at least 60%, at least 70%, and particularly of at least 80% or at least 90% of the crystal particles produced is within the range given by the mean diameter ± 10%. For example, if the mean diameter of the crystal particles produced is 25 µm, then the respective percentage of the crystal particles present in a given preparation have a diameter in the range between 22.5 µm and 27.5 µm.

Preferably, the water content of the crystalline monohydrate produced is in the range between 2.8% and 3.3% (w/w) (cf. above as well as the exemplary thermogravimetric analyses in **FIGURES 2**, **5**, and **8**).

In particular embodiments, the crystalline monohydrate of epirubicin hydrochloride is characterized by a particular a thermostability of less than 0.5% (w/w) doxorubicinone (i.e. the aglycone degradation product of epirubicin) being produced after incubation at 100°C for one month according to ICH guidelines (cf. *inter alia* ICH Guideline Q1A (R2)), as further given in **TABLE 8-1**.

In further particular embodiments, the crystalline monohydrate of epirubicin hydrochloride is further characterized by a particular powder X-ray diffraction pattern, as exemplified by the analyses shown in **FIGURES 3**, **6**, and **9**, wherein the diffraction pattern comprises characteristic peaks at average diffraction angles (2⊖) of 5.1°, 9.1°, 13.6°, 22.1°, 22.5°, and 24.0° (each ± 0.2°).

In specific embodiments, the crystalline monohydrate of epirubicin hydrochloride is further characterized by a particular differential scanning calorimetry (DSC) diagram, as exemplified by the analyses shown in **FIGURES 1**, **4**, and **7**, comprising a maximum intensity in the temperature range of 195°C to 205°C with an average peak (i.e. melting point) between 201°C and 204°C.

Finally, a further aspect of the present invention relates to the crystalline monohydrate of epirubicin hydrochloride as described herein above for use in the prevention and treatment of cancer. Particularly, the crystalline monohydrate of epirubicin hydrochloride is used for the treatment of breast cancer.

The invention is further described by the figures and the following examples, which are solely for the purpose of illustrating specific embodiments of this invention.

### EXAMPLES

### MATERIALS AND METHODS

Differential scanning calorimetry (DSC; for determining the melting point) was performed by precise sample weighing into alumina crucibles (70 µl, hermetically closed with an alumina lid) by using a calibrated ultra-micro balance. Measurement: Mettler TC11-TA-Processor with DSC 30 module or Mettler DSC 25 with silver-oven and ceramic sensor crystal and Mettler TC15A-TA-Controller (25°C to 250°C, 10°C/min). Purging gas: N₂, 80 ml/min, mass-flow controlled Calibration: Performed directly before the sample measurement with ultrapure Indium (In) as a reference material (temperature scale, heat-flow scale).

Coulometric Karl-Fischer titration (KFT; for determining the water content) was performed with an external drying oven (Metrohm 707 KF; operated at 140°C with N₂ purge). The samples were precisely weighed on an analytical balance (10⁻⁵ g).

Thermogrvimetric analysis (TG analysis; for determining the water content) was performed by precise sample weighing into alumina crucibles (100 µl, sealed with an alumina lid having a laser drilled 50 µm hole) by using a calibrated ultra-micro balance. Measurement: Mettler TGA/DSC1, large oven; gas control-box (purging gas: N₂, 80 ml/min, mass-flow controlled).

HPLC and GC analyses were performed according to standard protocols.

X-ray powder diffraction analysis was performed using a STOE-STADI P transmission diffractometer with the following setup: nCu-Kα₁ radiation (λ = 1.54056 A), U = 40 kV, I = 35 mA, primary beam monochromator (curved Ge(111)), linear position sensitive detector, slits: 1 mm, d = 8 mm, angle region: 2θ = 3 to 90°, step width Δ2θ = 0.02°, 25 s/0.2° step. The powder is originally filled between two mylar foils and then into the sample holder having a d = 8 mm mask.

### EXAMPLE 1

(1) 500.0 g of amorphous epirubicin hydrochloride (91.0% (w/w), as determined by HPLC analysis), were dissolved in 10 I of water. The water was evaporated on a rotor-evaporator under low pressure at a temperature of 40°C until a gel state of the solution was achieved.
(2) 1-butanol in an amount of 10 I was added to the solution and evaporation was continued until a solid precipitate was formed.
(3) Absolute ethanol in the amount of 5 I was added to the 1-butanol solution. The mixture was heated until the solid precipitate was re-dissolved. The water content of the 1-butanol/ ethanol solution was adjusted by means of evaporation to 8% to 10% (w/w) (as determined by Karl-Fisher titration). Subsequently, evaporation was stopped and the solution was stirred at a temperature of 75°C until a solid precipitate was formed.
(4) Crystals of epirubicin hydrochloride were collected by filtration, washed in 500 ml of acetone and air-dried at a temperature of 30°C to 35°C. 423 g of crystalline epirubicin hydrochloride were obtained (overall yield: 92.9%).

Functional characterization of the compound obtained:

| | |
|---|---|
| Melting point (DSC analysis): 201.4°C (cf. **FIG. 1**) | |
| Water content: | 3.11% (w/w) (KFT) |
| | 3.22% (w/w) (TG analysis; cf. **FIG. 2**) |
| | Calculated value for monohydrate: 3.01% (w/w)) |
| Epirubicin HCl content (HPLC analysis): 96.4% (w/w) | |
| Content of residual solvents (GC analysis): 0.26% (w/w) | |

X-ray powder diffraction analysis (cf. **FIG. 3**): characteristic peaks at diffraction angles (2⊖) of 5.1°, 9.1°, 13.6°, 22.1°, 22.5°, and 24.0°

The experimental data reveal that the compound obtained is epirubicin hydrochloride (HPLC) having a specifically defined crystalline structure (X-ray), a water content being indicative of a monohydrate form (KFT and TG), and being devoid of residual solvents (GC).

### EXAMPLE 2

(1) 500.0 g of amorphous epirubicin hydrochloride (content: 91.0% (w/w), as determined by HPLC analysis), were dissolved in 10 I of water. The water was evaporated on a rotor-evaporator under low pressure at a temperature of 40°C until a gel state of the solution was achieved.
(2) 1-butanol in an amount of 10 I was added to the solution and evaporation was continued until a solid precipitate was formed.
(3) 1-propanol in the amount of 5 I was added to the 1-butanol solution. The mixture was heated until the solid precipitate was re-dissolved. The water content of the n-butanol/1-propanol solution was adjusted by means of evaporation to 9% to 10% (w/w) (as determined by Karl-Fisher titration). Subsequently, evaporation was stopped and the solution was stirred at a temperature of 78°C until a solid precipitate was formed.
(4) Crystals of epirubicin hydrochloride were collected by filtration, washed in 500 ml of acetone and air-dried at a temperature of 30°C to 35°C. 435 g of crystalline epirubicin hydrochloride were obtained (overall yield: 95.6%).

Functional characterization of the compound obtained:

| | |
|---|---|
| Melting point (DSC analysis): 203.9°C (cf. **FIG. 4**) | |
| Water content: | 3.11% (w/w) (KFT) |
| | 3.17% (w/w) (TG analysis; cf. **FIG. 5**) |
| | Calculated value for monohydrate: 3.01% (w/w)) |
| Epirubicin HCl content (HPLC analysis): 96.1% (w/w) | |
| Solvent content (GC analysis): 0.23% (w/w) | |

X-ray powder diffraction analysis (cf. **FIG. 6**): characteristic peaks at diffraction angles (2⊖) of 5.1°, 9.1°, 13.6°, 22.1°, 22.5°, and 24.0°

The experimental data reveal that the compound obtained is epirubicin hydrochloride (HPLC) having a specifically defined crystalline structure (X-ray), a water content being indicative of a monohydrate form (KFT and TG), and being devoid of residual solvents (GC).

### EXAMPLE 3

(1) 500.0 g of amorphous epirubicin hydrochloride (content: 91.0% (w/w), as determined by HPLC analysis), were dissolved in 10 I of water. The water was evaporated on a rotor-evaporator under low pressure at a temperature of 40°C until a gel state of the solution was achieved.
(2) 1-butanol in an amount of 10 I was added to the solution and evaporation was continued until a solid precipitate was formed.
(3) A mixture of ethanol and 1-propanol (1:1 (v/v)) in the amount of 5 I was added to the 1-butanol solution. The mixture was heated until the solid precipitate was re-dissolved. The water content of the resulting solution was adjusted by means of evaporation to 8% to 10% (w/w) (as determined by Karl-Fisher titration). Subsequently, evaporation was stopped and the solution was stirred at a temperature of 75°C until a solid precipitate was formed.
(4) Crystals of epirubicin hydrochloride were collected by filtration, washed in 500 ml of acetone and air-dried at a temperature of 30°C to 35°C. 431 g of crystalline epirubicin hydrochloride were obtained (overall yield: 94.7%).

Functional characterization of the compound obtained:

| | |
|---|---|
| Melting point (DSC analysis): 201.6°C (cf. **FIG. 7**) | |
| Water content: | 3.11% (w/w) (KFT) |
| | 3.16% (w/w) (TG analysis; cf. **FIG. 8**) |
| | Calculated value for monohydrate: 3.01% (w/w)) |
| Epirubicin HCl content (HPLC analysis): 97.0 % (w/w) | |
| Solvent content (GC analysis): 0.21 % (w/w) | |

X-ray powder diffraction analysis (cf. **FIG. 9**): characteristic peaks at diffraction angles (2⊖) of 5.1°, 9.1°, 13.6°, 22.1°, 22.5°, and 24.0°

The experimental data reveal that the compound obtained is epirubicin hydrochloride (HPLC) having a specifically defined crystalline structure (X-ray), a water content being indicative of a monohydrate form (KFT and TG), and being devoid of residual solvents (GC).

The functional data obtained in Examples 1-3 show a very high degree of consistency being indicative of the production of the same compound.

### EXAMPLE 4 (COMPARATIVE EXAMPLE)

(1) 500.0 g of crystalline epirubicin hydrochloride were dissolved in 5 I of water. The water was evaporated on a rotor-evaporator under low pressure at a temperature of 40°C until a gel state of the solution was achieved.
(2) The gel solution of epirubicin hydrochloride was poured into 9 I of acetone.
(3) Precipitated crystals of epirubicin hydrochloride were collected by filtration, washed in 500 ml of acetone and air-dried at a temperature of 30°C to 35°C. 512 g of precipitated crystals were obtained. HPLC analysis confirmed the presence of epirubicin hydrochloride.
(4) Determination of the water content (calculated value for the trihydrate: 9.03% (w/w)

| | |
|---|---|
| Analysis: | 9.81% (w/w) (KFT) |
| | 9.85% (w/w) (TG analysis; cf. **FIG. 10**) |

The experimental data reveal that the crystalline epirubicin hydrochloride obtained has a water content being indicative of a trihydrate form.

### EXAMPLE 5

The crystalline monohydrate of epirubicin hydrochloride prepared according to Example 1 was exposed to a temperature of 25°C, 40°C, and 100°C, respectively, for the time period (in months) indicated. The testing was performed as described in the ICH guidelines (cf. *inter alia* ICH Guideline Q1A (R2)). The amount of the degradation product (i.e. doxorubicinone) produced as well as that of intact epirubicin hydrochloride (both in % (w/w)) was determined by HPCL analysis ("LT" = less than). The results are summarized in the **TABLE 5-1**.

The results demonstrate an exceptional high stability of the crystalline monohydrate produced, not only with regard to the overall shelf-life (stability over a time period of (at least) 5 years at a temperature of 25°C) but also with regard to thermostability (stability over a time period of (at least) 6 months at a temperature of 40°C).

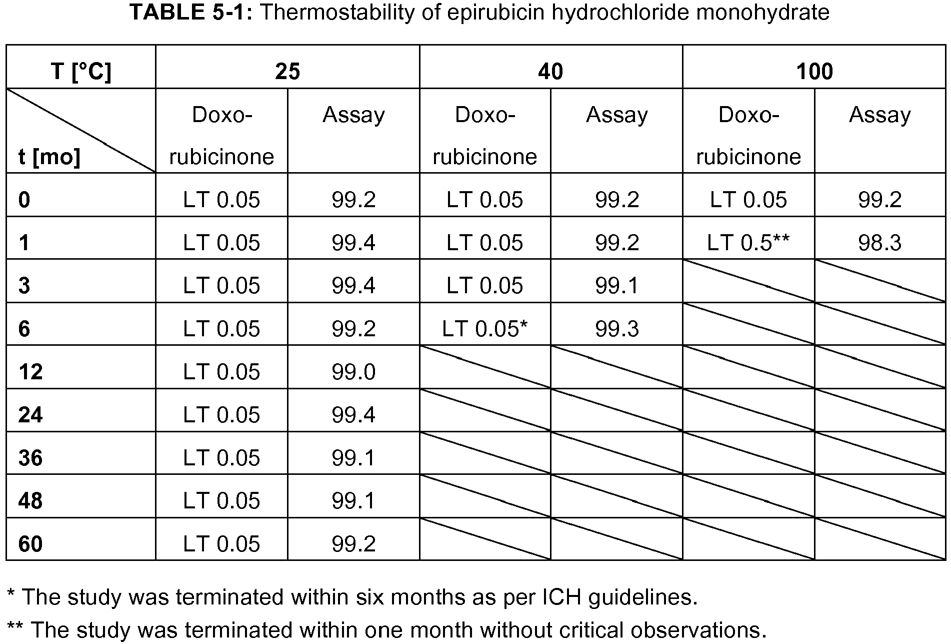

Additional analyses using the crystalline monohydrate of epirubicin hydrochloride prepared according to Examples 2 and 3, respectively, revealed almost identical results (not shown).

The same analysis was performed with the crystalline trihydrate of epirubicin hydrochloride prepared according to Example 4. The results are summarized in **TABLE 5-2**.

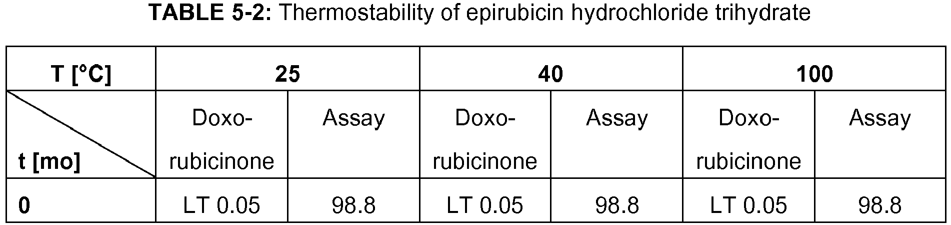

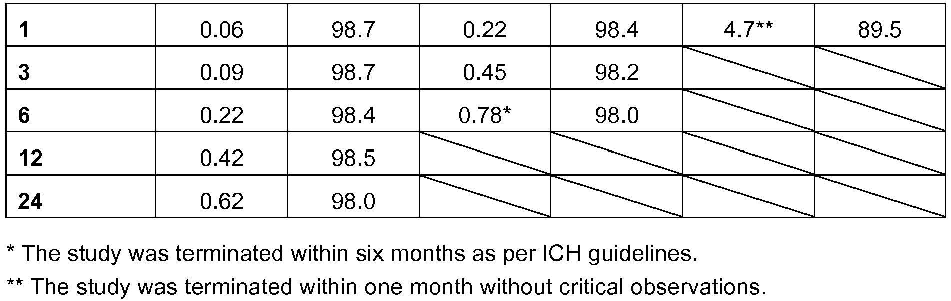

The data show significant degradation of the trihydrate form even within six months storage at a temperature of 25°C. Accumulation of the doxorubicinone aglycone drastically increases with elevated temperature. Hence, the superior properties of the crystalline epirubicin hydrochloride of the present invention are an inherent feature of the monohydrate form obtained.

### EXAMPLE 6

The crystalline monohydrate of epirubicin hydrochloride prepared according to Example 1 was exposed to a temperature of 25°C or 40°C and a relative humidity of 40-90%, respectively, for the time period (in days) indicated. The testing was performed as described in the ICH guidelines. The respective water contents of the samples (in % (w/w)) were measured by means of Karl-Fischer titration and the results summarized in **TABLE 6-1**.

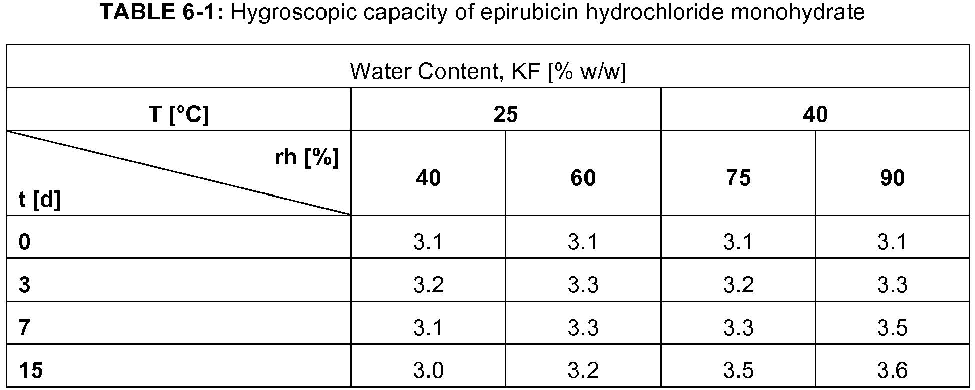

The experimental data show that the crystalline monohydrate of epirubicin hydrochloride produced does not exhibit any significant hygroscopic capacity. Even when exposed to 90% relative humidity and 40°C for 15 days the water absorption was surprisingly small.

Additional analyses using the crystalline monohydrate of epirubicin hydrochloride prepared according to Examples 2 and 3, respectively, revealed almost identical results (not shown). The same analysis was performed with the crystalline trihydrate of epirubicin hydrochloride prepared according to Example 4. The results are summarized in **TABLE 6-2**.

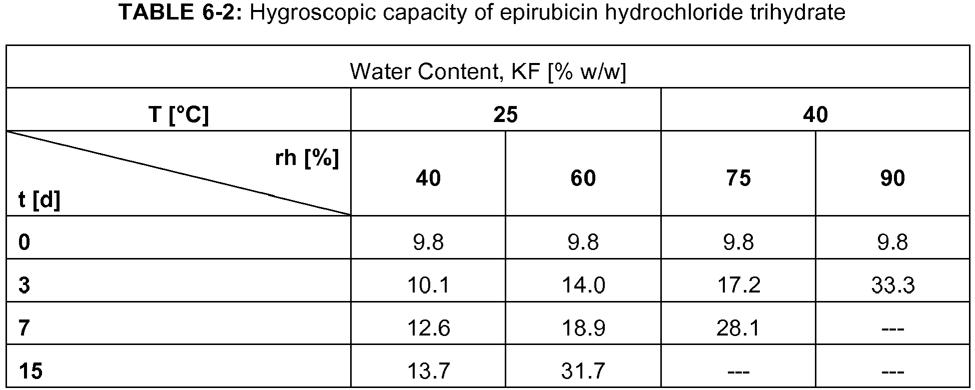

The data show a considerable extent of water absorption already after 3 days of exposure. Hence, again, the superior properties of the crystalline epirubicin hydrochloride of the present invention are an inherent feature of the monohydrate form obtained.

### EXAMPLE 7 (COMPARATIVE EXAMPLE)

Three samples of crystalline epirubicin hydrochloride being produced according to different methods were analyzed with respect to their respective particle size distributions by means of laser diffraction analysis.

**Sample 1** was prepared according to Example 1 of patent document WO 2012/163508 A1.

In brief, 9.0 g amorphous epirubicin hydrochloride were suspended in a mixture of 12 ml water, 258 ml 2-propanol, and 30 ml 1-butanol. This suspension was heated to 65°C, while stirring and left at this temperature for four hours. Then, the suspension was cooled stepwise to a temperature of 22°C. After the removal of the solvent contained in the suspension by filtration, the crystals were washed with acetone and dried for 24 hours under vacuum after the removal of acetone.

Laser diffraction analysis was performed in form of wet laser diffraction in acetone: 1750 rpm, 3 x 20 s cycle time, 60 s background time, without sonication, and 5 min stirring time using a Mastersizer 2000 Particle Size Analyzer (Malvern Instruments Ltd., Malvern, UK) according to the manufacturer's instructions.

**FIG. 11** shows the results of a representative analysis as well as exemplary wet dispersed microscopy images with 40-fold and 100-fold magnification, respectively.

The results demonstrate a variation of the particle sizes in the range between 1 µm and 80 µm with one peak at about 5 µm and a second peak at about 50 mm. The mean diameter based on the volume of the particles (also referred to as "D(v/0.5)") can be calculated to be 4.62 µm. The variation of the size of the particles is also directly visible under the microscope. Furthermore, the particles do not appear to be crystalline.

**Sample 2** was prepared according to Example 2 of patent document WO 2012/163508 A1.

In brief, 10.0 g amorphous epirubicin hydrochloride were dissolved in a mixture of 13 ml water and 13 ml 2-propanol, in order to prepare a solution containing epirubicin hydrochloride. This solution was then mixed with 33 ml 1-butanol and 274 ml 2-propanol. The produced mixture was heated to 65°C and left at this temperature for four hours, whereby epirubicin hydrochloride crystals were formed. Then, the obtained suspension was cooled stepwise to a temperature of 22°C. The solvent contained in the suspension was removed by filtration, and the crystals remaining as filter residue were washed with acetone. After removing the acetone, the crystals were dried for 24 hours under vacuum.

Laser diffraction analysis was performed as described above.

**FIG. 12** shows the results of a representative analysis as well as exemplary wet dispersed microscopy images with 40-fold and 100-fold magnification, respectively.

The results again demonstrate a considerable variation of the particle sizes in the range between 1 µm and 80 µm without a prominent single maximum peak. The D(v/0.5) value can be calculated to be 9.04 µm. The non-uniform size distribution of the particles is also directly visible under the microscope. Furthermore, the particles also do not appear to be crystalline.

**Sample 3** was prepared according to the present invention, as described in Example 3 above.

Laser diffraction analysis was performed as described above.

**FIG. 13** shows the results of a representative analysis as well as exemplary wet dispersed microscopy images with 40-fold and 100-fold magnification, respectively.

The results clearly illustrate a highly uniform distribution of the particle sizes with a single maximum peak at the D(v/0.5) value. The D(v/0.5) value can be calculated to be 25.25 µm. The vast majority of the crystal particles being present in the preparation has a size within the range given by the mean diameter ± 10%. Under the microscope, the particles really appear to be crystalline.

These data demonstrate that the method of the present invention provides for the production of a crystalline monohydrate of epirubicin hydrochloride having a highly uniform particle size distribution.

### EXAMPLE 8 (COMPARATIVE EXAMPLE)

Three samples of crystalline epirubicin hydrochloride being produced according to different methods were analyzed with respect to their thermostability under stress conditions (i.e. during storage at 100°C for one month).

**Sample S** was prepared according to the present invention, as described in Example 3 above. **Sample M1** was prepared according to Example 1 of WO 2012/163508 A1, as described in Example 7 above. Finally, **Sample M2** was prepared according to Example 2 of WO 2012/163508 A1, as described in Example 7 above.

The samples were exposed to a temperature of 100°C for the time period (in weeks) indicated. The testing was performed as described in the ICH Guideline Q1A (R2) (cf. above). The amount of the degradation product (i.e. doxorubicinone) produced as well as those of various impurities (i.e., doxorubicin and four different impurities of yet unknown identity; all given in % (w/w); "n.d." = not determined) was determined by HPCL analysis ("LT" = less than). The results are summarized in the **TABLE 8-1**.

In analogy to the thermostability data given above, the additional data obtained under stress conditions show a less pronounced degradation of the epirubicin hydrochloride prepared according to the present invention ("S") as compared to the samples prepared according to patent document WO 2012/163508 A1 ("M1" and "M2"). It is tempting to speculate that the decreased stability of the latter samples can be assigned to the greater specific surface area due to a higher molecular accessibility of amorphous materials as compared to crystalline states (cf. also Example 7). Furthermore, the epirubicin hydrochloride prepared according to the present invention ("S") has also a significantly reduced amount of impurities present in the sample (data shown in bold), thus providing further evidence for the superior properties of the epirubicin hydrochloride of the present invention, for example, with respect to product safety.

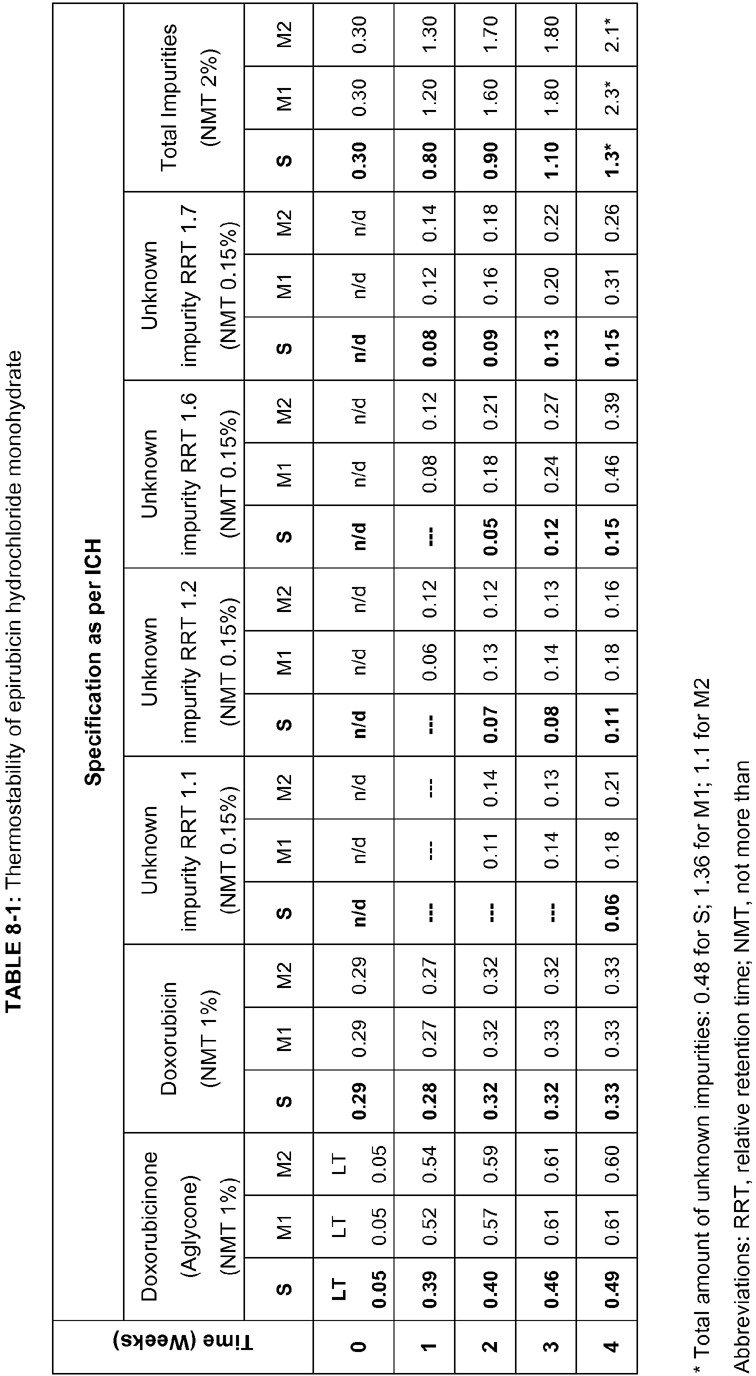

## Claims

1. Method for producing a crystalline monohydrate of epirubicin hydrochloride, comprising:
(a) adding at least a first solvent and at least a second solvent to epirubicin hydrochloride, wherein
(i) the first solvent is selected from the group consisting of 1-butanol, 2-butanol, isobutanol, tert-butanol, and mixtures thereof;
(ii) the second solvent is selected from the group consisting of 1-propanol, isopropanol, ethanol, and mixtures thereof; and
(iii) the volume ratio of the first solvent to the second solvent is in the range between 1:1 and 2:1 (v/v);
(b) adjusting in the solution obtained in step (a) the water content to an amount in the range between 8% and 11 % (w/w); and
(c) heating the solution obtained in step (b) to a temperature of 70°C to 90°C in order to allow crystallization;
wherein the crystalline monohydrate produced has water content in the range between 2.7% and 3.5% (w/w) and is devoid of residual solvents.

2. The method of claim 1, wherein the first solvent is 1-butanol and the second solvent is 1-propanol.

3. The method of claim 1 or 2, further comprising: heating the mixture of step (a) to a temperature of 40°C to 55°C until complete dissolution of the reaction mixture is accomplished.

4. The method of any one of claims 1 to 3, wherein the final concentration of epirubicin hydrochloride in the solution of step (b) is in the range between 10 g/l and 100 g/l.

5. The method of any one of claims 1 to 4, wherein the solution in step (c) is heated to a temperature of 75°C to 85°C.

6. The method of any one of claims 1 to 5, further comprising: seeding of the solution obtained in step (b) in order to induce crystallization.

7. The method of any one of claims 1 to 6, further comprising: purifying the crystals obtained in step (c).

8. The method of any one of claims 1 to 7, wherein the water content of the crystalline monohydrate of epirubicin hydrochloride is in the range between 2.8% and 3.3% (w/w).

9. Crystalline monohydrate of epirubicin hydrochloride, being produced by the method of any one of claims 1 to 8, wherein
(i) the mean diameter of the crystal particles produced is in the range between 20 µm and 50 µm; and
(ii) the particle size of at least 60%, and particularly of at least 80% of the crystal particles produced is within the range given by the mean diameter ± 20%.

10. The crystalline monohydrate of epirubicin hydrochloride of claim 9, wherein the particle size of at least 60%, and particularly of at least 80% of the crystal particles produced is within the range given by the mean diameter ± 10%.

11. The crystalline monohydrate of epirubicin hydrochloride of claim 9 or 10, further **characterized by** a thermostability resulting in the production of less than 0.5% (w/w) doxorubicinone after incubation at 100°C for one month according to ICH guidelines.

12. The crystalline monohydrate of epirubicin hydrochloride of any one of claims 9 to 11, further **characterized by** a powder X-ray diffraction pattern comprising peaks at average diffraction angles (2⊖) of 5.1°, 9.1°, 13.6°, 22.1°, 22.5°, and 24.0° (each ± 0.2°).

13. The crystalline monohydrate of epirubicin hydrochloride of any one of claims 9 to 12 for use in the prevention and treatment of cancer.

## Patentansprüche

1. Verfahren zur Herstellung eines kristallinen Monohydrats von Epirubicin-Hydrochlorid, das umfasst:
(a) Zugeben von zumindest einem ersten Lösungsmittel und zumindest einem zweiten Lösungsmittel zu Epirubicin-Hydrochlorid, wobei:
(i) das erste Lösungsmittel aus der Gruppe ausgewählt wird, die aus 1-Butanol, 2-Butanol, Isobutanol, tert-Butanol und Mischungen davon besteht;
(ii) das zweite Lösungsmittel aus der Gruppe ausgewählt wird, die aus 1-Propanol, Isopropanol, Ethanol und Mischungen davon besteht; und
(iii) das Volumenverhältnis des ersten Lösungsmittels zum zweiten Lösungsmittel im Bereich zwischen 1:1 und 2:1 (v/v) liegt;
(b) Einstellen des Wassergehalts in der in Schritt (a) erhaltenen Lösung auf eine Menge im Bereich zwischen 8% und 11% (w/w); und
(c) Erhitzen der in Schritt (b) erhaltenen Lösung auf eine Temperatur von 70°C bis 90°C, um eine Kristallisation zu ermöglichen;
wobei das hergestellte kristalline Monohydrat einen Wassergehalt im Bereich zwischen 2.7% und 3.5% (w/w) besitzt und frei von Restlösungsmitteln ist.

2. Verfahren gemäß Anspruch 1, wobei das erste Lösungsmittel 1-Butanol ist, und das zweite Lösungsmittel 1-Propanol ist.

3. Verfahren gemäß Anspruch 1 oder 2, das ferner umfasst: Erhitzen der Mischung aus Schritt (b) auf eine Temperatur von 40°C bis 50°C, bis eine vollständige Auflösung des Reaktionsgemischs erreicht ist

4. Verfahren gemäß einem der Ansprüche 1 bis 3, wobei die Endkonzentration von Epirubicin-Hydrochlorid in der Lösung in Schritt (b) im Bereich zwischen 10 g/l und 100 g/l liegt.

5. Verfahren gemäß einem der Ansprüche 1 bis 4, wobei die Lösung in Schritt (c) auf eine Temperatur von 75°C bis 85°C erhitzt wird.

6. Verfahren gemäß einem der Ansprüche 1 bis 5, das ferner umfasst: Seeding der in Schritt (b) erhaltenen Lösung, um die Kristallisation zu induzieren.

7. Verfahren gemäß einem der Ansprüche 1 bis 6, das ferner umfasst: Reinigen der in Schritt (c) erhaltenen Kristalle.

8. Verfahren gemäß einem der Ansprüche 1 bis 7, wobei der Wassergehalt des kristallinen Monohydrats von Epirubicin-Hydrochlorid im Bereich zwischen 2.8% und 3.3% (w/w) besitzt

9. Kristallines Monohydrat von Epirubicin-Hydrochlorid, hergestellt durch das Verfahren gemäß einem der Ansprüche 1 bis 8 hergestellt wird, wobei:
(i) der mittlere Durchmesser der hergestellten Kristallpartikel im Bereich zwischen 20 µm und 50 µm liegt; und
(ii) die Partikelgröße von zumindest 60%, und insbesondere von zumindest 80% der hergestellten Partikel innerhalb des Bereichs des mittleren Durchmessers ± 20% liegt.

10. Kristallines Monohydrat von Epirubicin-Hydrochlorid gemäß Anspruch 9, wobei die Partikelgröße von zumindest 60%, und insbesondere von zumindest 80% der hergestellten Partikel innerhalb des Bereichs des mittleren Durchmessers ± 10% liegt.

11. Kristallines Monohydrat von Epirubicin-Hydrochlorid gemäß Anspruch 9 oder 10, das ferner durch eine Thermostabilität charakterisiert ist, die nach Inkubation bei 100°C für einen Monat gemäß ICH Richtlinien die Erzeugung von weniger als 0.5% (w/w) Doxorubicinon zur Folge hat.

12. Kristallines Monohydrat von Epirubicin-Hydrochlorid gemäß einem der Ansprüche 9 bis 11, das ferner durch ein Pulverröntgenbeugungsmuster charakterisiert ist, das Peaks bei durchschnittlichen Beugungswinkeln (2⊖) von 5.1°, 9.1°, 13.6°, 22.1°, 22.5° und 24.0° (jeweils ± 0.2°) aufweist.

13. Kristallines Monohydrat von Epirubicinhydrochlorid gemäß einem der Ansprüche 9 bis 12 zur Verwendung bei der Vorbeugung und Behandlung von Krebs.

## Revendications

1. Procédé de production d'un monohydrate cristallin de chlorhydrate d'épirubicine, comprenant:
(a) l'addition d'au moins un premier solvant et au moins un second solvant au chlorhydrate d'épirubicine, dans lequel
(i) le premier solvant est choisi dans le groupe consistant en 1-butanol, 2-butanol, isobutanol, tert-butanol et leurs mélanges;
(ii) le second solvant est choisi dans le groupe consistant en 1-propanol, isopropanol, éthanol et leurs mélanges; et
(iii) le rapport volumique du premier solvant au second solvant est dans la plage entre 1:1 et 2:1 (v/v);
(b) ajustement dans la solution obtenue dans l'étape (a) de la teneur en eau à une quantité dans la plage entre 8% et 11% (p/p); et
(c) chauffage de la solution obtenue dans l'étape (b) à une température de 70°C à 90°C pour permettre la cristallisation;
dans lequel le monohydrate cristallin produit a une teneur en eau dans la plage entre 2.7% et 3.5% (p/p) et est dépourvu de solvants résiduels.

2. Procédé selon la revendication 1, dans lequel le premier solvant est le 1-butanol et le second solvant est le 1-propanol.

3. Procédé selon la revendication 1 ou 2, comprenant en outre: chauffage du mélange de l'étape (a) à une température de 40°C à 55 C jusqu'à l'achèvement de la dissolution complète du mélange réactionnel.

4. Procédé selon l'une quelconque des revendications 1 à 3, dans lequel la concentration finale en chlorhydrate d'épirubicine dans la solution de l'étape (b) se situe dans la plage entre 10 g/l et 100 g/l.

5. Procédé selon l'une quelconque des revendications 1 à 4, dans lequel la solution dans l'étape (c) est chauffée à une température de 75°C à 85°C.

6. Procédé selon l'une quelconque des revendications 1 à 5, comprenant en outre:
l'ensemencement de la solution obtenue à l'étape (b) pour induire la cristallisation.

7. Procédé selon l'une quelconque des revendications 1 à 6, comprenant en outre: la purification des cristaux obtenus à l'étape (c).

8. Procédé selon l'une quelconque des revendications 1 à 7, dans lequel la teneur en eau du monohydrate cristallin du chlorhydrate d'épirubicine est dans la plage entre 2.8% et 3.3% (p/p).

9. Monohydrate cristallin de chlorhydrate d'épirubicine, produit par le procédé selon l'une quelconque des revendications 1 à 8, dans lequel
(i) le diamètre moyen des particules cristallines produites est dans la plage entre 20 µm et 50 µm; et
(ii) la granulométrie d'au moins 60% et en particulier d'au moins 80% des particules cristallines produites est dans la plage donnée par le diamètre moyen ± 20%.

10. Monohydrate cristallin de chlorhydrate d'épirubicine selon la revendication 9, **caractérisé en ce que** la granulométrie d'au moins 60%, et en particulier d'au moins 80% des particules cristallines produites, se situe dans la plage donnée par le diamètre moyen ± 10%.

11. Monohydrate cristallin de chlorhydrate d'épirubicine selon la revendication 9 ou 10, **caractérisé en outre par** une thermostabilité conduisant à la production de moins de 0.5% (p/p) de doxorubicinone après incubation à 100°C pendant un mois selon les directives de l'ICH.

12. Monohydrate cristallin de chlorhydrate d'épirubicine selon l'une quelconque des revendications 9 à 11, **caractérisé en outre par** un diagramme de diffraction des rayons X de poudre comprenant des pics à des angles de diffraction moyens (2⊖) de 5.1°, 9.1°, 13.6°, 22.1°, 22.5°, et 24.0° (chacun ± 0.2°).

13. Monohydrate cristallin de chlorhydrate d'épirubicine selon l'une quelconque des revendications 9 à 12, destiné à être utilisé dans la prévention et le traitement du cancer.
